(19) Europäisches Patentamt
European Patent Office
Office européen des brevets

(11) EP 1 545 564 B1

(12) **EUROPÄISCHE PATENTSCHRIFT**

(45) Veröffentlichungstag und Bekanntmachung des Hinweises auf die Patenterteilung:
**05.09.2007 Patentblatt 2007/36**

(21) Anmeldenummer: **03753479.9**

(22) Anmeldetag: **30.09.2003**

(51) Int Cl.:
***A61K 31/728*** *(2006.01)* ***A61K 45/06*** *(2006.01)*
***A61P 19/02*** *(2006.01)*

(86) Internationale Anmeldenummer:
**PCT/EP2003/010822**

(87) Internationale Veröffentlichungsnummer:
**WO 2004/032943 (22.04.2004 Gazette 2004/17)**

(54) **KOMBINATIONSPRÄPARAT AUS HYALURONSÄURE UND LIDOCAIN UND DESSEN VERWENDUNG**

COMBINATION PREPARATION OF HYALURONIC ACID AND LIDOCAINE AND THE USE THEREOF

PREPARATION COMBINEE D'ACIDE HYALURONIQUE ET LIDOCAINE ET UTILISATION DE CETTE PREPARATION

(84) Benannte Vertragsstaaten:
**AT BE BG CH CY CZ DE DK EE ES FI FR GB GR HU IE IT LI LU MC NL PT RO SE SI SK TR**

(30) Priorität: **04.10.2002 DE 10246340**

(43) Veröffentlichungstag der Anmeldung:
**29.06.2005 Patentblatt 2005/26**

(73) Patentinhaber: **Wohlrab, David**
**06118 Halle (DE)**

(72) Erfinder: **Wohlrab, David**
**06118 Halle (DE)**

(74) Vertreter: **Pfenning, Meinig & Partner GbR**
**Patent- und Rechtsanwälte**
**Theresienhöhe 13**
**80339 München (DE)**

(56) Entgegenhaltungen:
**US-A- 5 972 326** **US-B1- 6 224 857**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 545 564 B1

**Beschreibung**

Die Erfindung betrifft die Verwendung eines Kombinationspräparats bestehend aus Hyaluronsäure und/oder deren Salzen und Lidocain sowie gegebenenfalls weiteren Zusatzstoffen, wobei Hyaluronsäuve und/oder deren Salze und Lidocain in chemisch oder physikalisch gebundener Form verwendet werden. Diese Kombinationspräparate finden Verwendung für die medizinische Behandlung von degenerativen und traumatischen Erkrankungen aller Gelenke, zur Behandlung von Gelenkknorpel- und Knorpelknochendefekten sowie Meniskus- und Bandscheibenläsionen wie z.B. Arthrose, Gelenkrheumatismus, Osteochondrosis dissecanz, flake fractures, Meniskusläsionen.

Der chemische Name für Hyaluronsäure ist Hyaluronan. Seine chemische Struktur entspricht der Formel

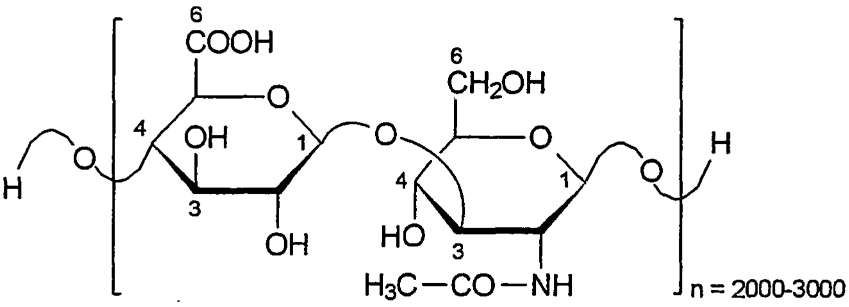

Ungeachtet der positiven klinischen Erfahrungen mit hochmolekularer Hyaluronsäure bzw. deren Salzen (Momasse > $1x10^6$ Dalton) ist die Kenntnis über den Wirkmechanismus unvollständig. Der bisherige Wissensstand weist intraartikulär applizierte Hyaluronsäure als ein Schmier- und Gleitmittel aus (A. Lussier et al. (1996); J Rheumatol. 23, 1579-1585; D. Scale et al.(1994); Current Therapeutic Research. 55, 220-232; M. Wobig et al. (1998) Clinical Therapeutics. 20, 410-4.23). Des Weiteren wurde nachgewiesen, daß Hyaluronsäure intraartikulär entzündungshemmende Eigenschaften besitzt (K.W.Marshall (1997) Today's Therapeutic Trends. 15, 99-108; K.W.Marshall (2000) Curr. Opin.Rheumatol. 12, 468-474).

Die Arthrose beginnt mit einer initialen Schädigung des Knorpelgewebes aufgrund verschiedener Ursachen. Dies hat eine reaktive Synovialitis zur Folge, welche ihrerseits sowohl pathologische Veränderungen der Synovialflüssigkeit, d.h. Abnahme der Konzentration und des Molekulargewichtes der Hyaluronsäure, sowie die Freisetzung von Entzündungsmediatoren bewirkt. Dies führt zu einer sekundären Knorpelschädigung und damit letztendlich zur Arthrose, welche neben dem Knorpelgewebe auch alle anderen Gelenkstrukturen betrifft (J.P.Pelletier et al. (1993) J Rheumatol. 20, 19-24).

Es ist bekannt, daß intraartikulär applizierte Hyaluronsäure zur Verbesserung der Gelenkbeweglichkeit, zur Schmerzreduktion, zur Hemmung der Entzündungsprozesse und unter in vitro Bedingungen zur Steigerung der Chondrozyten-Proliferation führt (K.Kawasaki et al. (1999) Cell Physiol. 179, 142-148; D. Wohlrab et al. (2000) hylan news. 2; 2-5).

Ausgehend hiervon war es Aufgabe der vorliegenden Erfindung, ein Kombinationspräparat bereitzustellen, daß in vielfältiger Form applizierbar ist und bei dem die Wirkstoffe gezielt retardiert freigesetzt werden können.

Diese Aufgabe wird durch die Verwendung des Kombinationspräparats mit den Merkmalen des Anspruchs 1 gelöst.

Erfindungsgemäß wird die Verwendung gemäß Anspruch 1 bereit gestellt

Es wurde festgestellt, daß durch die erhebliche Molekülgröße der Hyaluronsäure ($1-6x10^6$ Da) diese mehrfach gespalten werden muss; bevor sie den intraartikulären Raum verlassen und abgebaut bzw. in Knorpelgewebe eingebaut werden kann. Diese Spaltungsprozesse nehmen in Abhängigkeit von der Molmasse der Hyaluronsäure Stunden bis mehrere Tage in Anspruch.

Aufgrund dieser im Vergleich zu anderen niedermolekularen Substanzen, wie z.B. Lidocain verlängerten intraartikulären Verweildauer eignet sich hochmolekulare Hyaluronsäure, deren Salze als Träger für Substanzen, welche ohne derartige Bindung an ein Trägermolekül eine deutlich verkürzte intraartikuläre Verweildauer und damit eine sehr kurze Wirkdauer aufweisen.

Als galenische Formulierung kommen folgende aus dem Stand der Technik bekannten Formulierungen in Frage, wie intraartikulär oder intradiscal anwendbare galenische Formulierungen.

Der pH-Wert der Formulierung ermöglicht dabei eine optimale Bindung zwischen den beiden Wirkstoffen und die Freigabe des Lidocains kann über die Veränderung des pH-Wertes des Umgebungsmediums gesteuert werden.

Vorzugsweise ist Hyalwonsäure in dem Kombinationspräparat in einer Konzentration zwischen 0,001 und 5

Gew.-% oder bevorzugt zwischen 0,2 und 2,0 Gew.-% enthalten. Lidocain ist vorzugsweise in einer Konzentration zwischen 0,001 und 20 Gew.%, bevorzugt zwischen 0,001 und 5,0 Gew.-% enthalten. Weiterhin können in dem Kombinationspräparat weitere Zusatzstoffe enthalten sein. Hierzu zählen beispielsweise Substanzen mit Radikalfänger-Eigenschaften, insbesondere Tocopherolderivate oder Ascorbinsäurederivate. Des Weiteren können Substanzen des hyalinen Knorpelgewebes eingesetzt werden, insbesondere Glucosaminsulfatderivate oder Chondroitinsulfatderivate. Weiterhin können Substanzen mit steroidaler und corticosteroidaler Wirkung eingesetzt werden, insbesondere Glucocorticoide. Als Zusatzstoffe kommen weiterhin nicht-steroidale Antiphlogistika, die auch als Anti-Rheumatika bezeichnet werden, insbesondere Indometacin, Dichlorphenac oder Salicylsäurederivate und Analgetica, insbesondere Oxicame, Anilin- oder Anthranilsäurederivate in Frage. Das Kombinationspräparat kann als Zusatzstoff ebenfalls Substanzen mit hemmender Wirkung auf die Prostaglandinsynthese, insbesondere Lipoxygenase-Hemmer, Cyclooxygenase-Hemmer und Phospholipase-A2-Hemmer. Ebenso kommen als Zusatzstoffe Wachstumsfaktoren, insbesondere Retinol oder Bone Morphogenetic Proteins (BMP's), Vitamine, insbesondere Vitamin A, C, B12 oder Biotin, Antioxidantien, insbesondere Flavonoide oder Glutathion, und Substanzen mit wasserbindenden Eigenschaften, insbesondere Harnstoff oder Arginin in Frage.

Die Anwendung des Kombinationspräparats kann sowohl am Menschen als auch an Tieren erfolgen. Die erfindungsgemäßen Kombinationspräparate können in der Human- und Veterinärmedizin angewendet werden.

Die Anwendungsgebiete der Kombinationspräparate betreffen die human- und veterinärmedizinische Therapie, Prophylaxe und/oder Metaphylaxe von degenerativen oder traumatischen Gelenkerkrankungen und Gelenkfunktionsstörungen, Gelenkknorpel- und Knorpelknochendefekten, Meniskus- und Bandscheibenerkrankungen. Hierzu zählen beispielsweise die Steigerung der Chondrozyten-Proliferation, die Stabilisierung und/oder Regeneration von Gelenkstrukturen, insbesondere des Gelenkknorpels und der Menisci, die Steigerung der Gelenkbeweglichkeit und die Hemmung von Entzündungsprozessen.

Die Erfindung soll anhand der folgenden Beispiele und Figuren erläutert werden, ohne sie darauf zu beschränken.

<u>Beispiel 1:</u>

Physiologische Verträglichkeit der erfindungsgemäßen galenischen Formulierungen

Herstellung:

Lidocainhydrochlorid (Universitätsapotheke der Martin-Luther-Universität Halle-Wittenberg) und Hyaluronsäure (Aqua Biochem, Dessau) (MG $1,5x10^6$ Da)lagen primär in Pulverform vor. Zur Herstellung von 2%-igen Stammlösungen wurden entsprechende Mengen in RPMI-Medium (Seromed, Berlin) gelöst und anschließend steril filtriert. Zur Herstellung eines Lidocain-Hyaluronsäure-Gemisches wurden diese Stammlösungen zu gleichen Teilen vermischt. Die Substanzzugabe zur Zellkultur erfolgte am 10. Kulturtag beim Mediumwechsel. Hier wurden entsprechende Mengen der Testsubstanzen (Stammlösungen) zugegeben, so daß eine jeweilige Endkonzentration von $5x10^{-5}$ mmol/l erreicht wurde.

Präparation des biologischen Materials:

Die Untersuchungen erfolgten an humanen Chondrozyten, welche aus arthrotisch verändertem Kniegelenksknorpel isoliert wurden. Das Knorpelgewebe entstammte den bei der Implantation von Knietotalendoprothesen resizierten femoralen Gelenkflächen. Es wurde ausschließlich arthrotisch verändertes Knorpelgewebe von drei verschiedenen Spendern ohne bekannte relevante Nebenerkrankungen, insbesondere ohne rheumatoide Arthritis, verwandt.

Die intraoperativ gewonnenen Knochen-Knorpelfragmente wurden zunächst in steriles L15 Medium (Seromed, Berlin) als Transportmedium überführt. Anschließend erfolgte unter sterilen Bedingungen die Ablösung des Knorpelgewebes vom subchondralen Knochen mittels Skalpell sowie eine scharfe Durchtrennung des Gewebes in ca. $1mm^3$ große Stücke. Die enzymatische Isolierung der Chondrozyten aus den Knorpelstücken erfolgte mittels Pronase und Kollagenase A (Boehringer Mannheim) über eine Zeitspanne von 16 Stunden.

Versuchsbedingungen:

Die isolierten Chondrozyten wurden in 24-er well Platten in RPMI-Medium (Seromed, Berlin) unter Zusatz verschiedener Antibiotika bei 37°C und 5% Kohlendioxid im Brutschrank als Monolayerkultur kultiviert. Der Mediumwechsel erfolgte alle 2 Tage. Nach 10 Kulturtagen erfolgte letztmalig ein Mediumwechsel und hierbei die Zugabe der jeweiligen Testsubstanzen, welche im Kulturmedium gelöst wurden. Zusätzlich wurde jeweils eine unbehandelte Chondrozytenpopulation als Kontrolle mitgeführt.

Versuchsdurchführung:

Die Messung des $^3$H-Thymidineinbaus als Maß für die DNA-Syntheseleistung erfolgte 24, 48 bzw. 72 Stunden nach Substanzzugabe. Am Ende der Kulturdauer wurde zu der Zellkultur je well 20 µl $^3$H-methyl-Thymidin (spezifische Aktivität 60,3 Ci/mmol; American Radiolabeled Chemicals Inc., St. Louis, USA) zugegeben. Zwei Stunden nach $^3$H-Thymidinzugabe wurde das Medium aus den Kammern mit Hilfe eines Cell Harvesters (Berthold GmbH, Bad Wildbad) abgesaugt. Jede Kulturkammer wurde mit 200 µl Trypsin beschickt und nach 20 Minuten wurde die Zellsuspension über einen Filter abgesaugt. Anschließend erfolgte die Messung der Radioaktivität der Zellen im Filterpapier mit Hilfe eines Flüssigkeitsszintillationszählers (WINSPECTRAL 1414, Wallace-ADL GmbH, Freiburg, Deutschland).

Die Ergebnisse für die physiologische Verträglichkeit der erfindungsgemäßen galenischen Formulierungen sind in Fig. 1 dargestellt.

Fig. 1 zeigt den Einfluss von Hyaluronsäure (Hys) (1, $5\times10^6$ Da, $5x10^{-5}$ mmol/l), Lidocain (Lido) ($5x10^{-5}$ mmol/1) und Hyaluronsäure-Lidocain-Gemisch (Hys+Lido) (je $5x10^{-5}$ mmol/1) auf den $^3$H-Thymidineinbau von in vitro kultivierten humanen Chondrozyten (N=3) nach 48 h Inkubationszeit. Jeder Messwert ist der Mittelwert von 8 Einzelmessungen.

Beispiel 2:

Beeinflussung der Proliferation humaner Chondrozyten durch Lidocain

Herstellung:

Lidocainhydrochlorid (Universitätsapotheke der Martin-Luther-Universität Halle-Wittenberg) lag primär in Pulverform vor. Dieses wurde in entsprechender Menge in RPML-Medium (Seromed, Berlin) gelöst, so daß eine Endkonzentration von 0,1 mmol/l Lidocain vorlag. Anschließend erfolgte die Sterilfiltration. Die Substanzzugabe zur Zellkultur erfolgte ab dem 2. Kulturtag bei jedem Mediumwechsel. Hier wurden entsprechende Mengen der Testsubstanzen (Stammlösungen) zugegeben, so daß eine jeweilige Endkonzentration von $5x10^{-5}$ mmol/l erreicht wurde.

Präparation des biologischen Materials

Die Präparation des Knorpelgewebes und der daraus isolierten Chondrozyten erfolgte analog den in Beispiel 1 dargestellten Methoden.

Versuchsbedingungen:

Die isolierten Chondrozyten wurden in 24-er well Platten in RPMI-Medium (Seromed, Berlin) unter Zusatz verschiedener Antibiotika bei 37°C und 5% Kohlendioxid im Brutschrank als Monolayerkultur kultiviert. Der Mediumwechsel erfolgte alle 2 Tage. Ab dem ersten Mediumwechsel erfolgte die Zugabe des Lidocains im Zellkulturmedium in einer Konzentration 0,1 mmol/l, Zusätzlich wurde jeweils eine unbehandelte Chondrozytenpopulation als Kontrolle mitgeführt. Die Kulturdauer betrug 6, 12 bzw. 18 Tage.

Versuchsdurchführung:

Die Messung des $^3$H-Thymidineinbaus als Maß für die DNA-Syntheseleistung erfolgte am jeweiligen Ende der Kulturdauer analog dem im Beispiel 1 dargestellten Verfahren. Die Ergebnisse der Untersuchungen sind in Fig. 2 dargestellt.

Fig. 2 zeigt den Einfluß von Lidocain (0,1 mmol/1) auf den $^3$H-Thymidineinbau von in vitro kultivierten humanen Chondrozyten (N=6). Substanzzugabe am 2. Kulturtag. Jeder Messwert ist der Mittelwert von 8 Einzelmessungen

Beispiel 3

Die optimale Bindung des Lidocains an Hyaluronsäure und/oder den physiologisch verträglichen Salzen der Hyaluronsäure

Tabelle 1 zeigt nachfolgend den Anteil an freiem Lidocain bei unterschiedlichen Lidocainkonzentrationen (Konz. Hyaluronsäure=0,05%)

| Konz. Lidocain [%] | | 0,025 | 0,05 | 0,01 | 0,2 | 0,3 | 0,4 | 0,5 |
|---|---|---|---|---|---|---|---|---|
| Freies Lidocain [%] | pH 6,9 | 54 | 55 | 70 | 71 | 87 | 89 | 93 |
| | pH 7,9 | 34 | 81 | 79 | 82 | 89 | 91 | 105 |

Experimentelle Bedingungen:

3D CE system der Fa. Hewlett Packard mit fused silica Kapillare 40,0 (48,5) cm mit Innendurchmesser 50 $\mu$m, Temp.: 25°C, Druckinjektion: 50 mbar x sec, Spannung: +30 kV, UV-Detektion: kathodenseitig bei $\lambda$ = 195 nm und 200 nm, Injektionszeit: 200 sec. Durch die Injektionszeit wurden 7,5 cm der Kapillare mit der Probe gefüllt, um eine optimale Trennung der Peaks zu erreichen.

Anhand der elektrophoretischen Frontalanalyse konnte gezeigt werden, daß eine Wechselwirkung zwischen Hyaluronsäure (Hys) und Lidocain auftritt. Wenn gleiche prozentuale Anteile von Hys und Lidocain bzw. wenn prozentual weniger Lidocain als Hys vorliegt, wird der größte Anteil an Lidocain an Hys gebunden. Der Mechanismus der Wechselwirkung beruht auf einer Einlagerung des Lidocains in die helixartigen Knäuel der Hys, da auch bei pH-Wert 7,9, wenn Lidocain zur Hälfte undissoziiert vorliegt (pKs-Wert = 7,9 in Anwesenheit von Hys). Ausserdem sind ionische Bindungen an der Wechselwirkung beteiligt, da bei pH 6,9, wenn Lidocain vollständig dissoziiert vorliegt, weniger freies Lidocain detektiert werden konnte (außer bei einer Lidocainkonz. = 0,025%).

Beispiel 4

Retardierte Freisetzung des Lidocains aus Formulierungen, die Hyaluronsäure und/oder physiologisch verträgliche Salze der Hyaluronsäure enthalten

Tabelle 2 zeigt nachfolgend den Flux des Lidocains durch eine Dialysemembran mit und ohne Hyaluronsäure (Hys) im Donorkompartiment

| pH-Wert | | 3,1 | 6,0 | 6,5 | 6,9 | 7,7 | 9,0 |
|---|---|---|---|---|---|---|---|
| Flux [mg $h^{-1}$ $cm^{-2}$] | Lidocain | 0,32 | 0,355 | 0,315 | 0,42 | 0,35 | 0,09 |
| | Lidocain + Hys | 0,27 | 0,256 | 0,234 | 0,27 | 0,23 | 0,04 |
| Differenz [%] $Flux_{Lidocain}$ - $FIUX_{Lidocain+Hys}$ | | 15.6 | 27,8 | 25,8 | 35,7 | 34,3 | 55,6 |

Experimentelle Bedingungen: $cm^3$

Diffusionszelle mit Diffusionsfläche (A) = 15,9 $cm^3$ und einer Natrium-Cellulose-Xanthogenat-(Nephrophan) Dialyse Membran, Volumen (V) des Donor-(DK) und des Akzeptorkompartiments (AK) = 20 ml, Diffusionszeit = 4 h, Temp.: 37°C, Konzentration der Hys im Donorkompartiment = 0,25% und die Anfangskonzentration des Lidocains im Donorkompartiment = 0,05%.

Berechnung des Fluxes:

$$\mathrm{Flux} = \frac{C_{AK}\, V_{AK}}{A\, t}.$$

Dabei sind:

$C_{AK=}$ Konzentration des Lidocains im AK und
t =Diffusionszeit.

Anhand der Resultate, die in der Dialysezelle erhalten wurden, zeigte sich, daß der Flux des Lidocains durch diese Porenmembran bei Anwesenheit der Hys im Donorkompartiment erheblich reduziert wurde. Am stärksten ausgeprägt ist der Effekt bei pH = 9,0, dort liegt Lidocain weitgehend undisoziiert vor. Dies bestätigt die Resultate, die in Beispiel 3 beschrieben wurden, daß der Mechanismus der Wechselwirkung zwischen Lidocain und Hys auf einer Einlagerung des Lidocains in die helixartigen Knäuel der Hys beruht. Aber auch bei pH-Werten zwischen 6,9 und 7,7 ist eine starke Reduzierung des Lidocainfluxes zu beobachten. Das bestätigt, daß auch ionogene Bindungen and der Interaktion zwischen Lidocain und Hys beteiligt sind. Verschiebt man den pH-Wert in den sauren Bereich z.B. nach pH = 3,1, dort liegt die Hys weitgehend undissoziiert vor, wird der Lidocainflux weniger stark reduziert. Das zeigt deutlich, daß auch ionogene Bindungen an der Wechselwirkung beteiligt sind.
Insgesamt kann festgestellt werden, daß ein starker Retardeffekt hinsichtlich der Freisetzung des Lidocains aus dem Lidocain-Hys-Komplex erzielt werden kann. Dadurch kann die Wirkung des Lidocains in biologischen Systemen (z.B. im Kniegelenk) erheblich verlängert werden.

## Patentansprüche

1. Verwendung von Hyaluronsäure und/oder deren Salzen in Kombination mit Lidocain sowie gegebenenfalls weiteren Zusatzstoffen als intraartikuläre oder intradiscale Formulierung zur Herstellung eines Präparats zur human- und veterinärmedizinischen Therapie, Prophylaxe und/oder Metaphylaxe von degerenativen oder traumatischen Gelenkerkrankungen und Gelenkfunktionsstörungen, wobei Hyaluronsäure und/oder deren Salze und Lidocain in chemisch oder physikalisch aneinander gebundener Form verwendet werden, und dass Lidocain retardiert freigesetzt wird.

2. Verwendung nach Anspruch 1 zur human- und veterinärmedizinischen Therapie, Prophylaxe und/oder Metaphylaxe von Gelenkknorpel- und Knorpelknochendefekten.

3. Verwendung nach mindestens einem der Ansprüche 1 und 2 zur human- und veterinärmedizinischen Therapie, Prophylaxe und/oder Metaphylaxe von Meniskus- und Bandscheibenerkrankungen.

4. Verwendung nach mindestens einem der Ansprüche 1 bis 3 zur Stabilisierung und/oder Regeneration von Gelenkstrukturen, insbesondere des Gelenkknorpels und des Meniskus.

5. Verwendung nach mindestens einem der Ansprüche 1 bis 4, **dadurch gekennzeichnet, dass** Hyaluronsäure und/oder dessen Salze in einer Konzentration zwischen 0,001 und 5 Gew.-% verwendet wird.

6. Verwendung nach mindestens einem der Ansprüche 1 bis 5, **dadurch gekennzeichnet, dass** Hyaluronsäure und/oder dessen Salze in einer Konzentration zwischen 0,2 und 2 Gew.-% verwendet wird.

7. Verwendung nach mindestens einem der Ansprüche 1 bis 6, **dadurch gekennzeichnet, dass** Lidocain in einer Konzentration zwischen 0,001 und 20 Gew.-% verwendet wird.

8. Verwendung nach mindestens einem der Ansprüche 1 bis 7, **dadurch gekennzeichnet, dass** Lidocain in einer Konzentration zwischen 0,001 und 5,0 Gew.% verwendet wird.

9. Verwendung nach einem der Ansprüche 1 bis 8, **dadurch gekennzeichnet, dass** als Zusatzstoffe Radikalfänger ausgewählt aus Tocopherolderivaten und/oder Ascorbinsäurederivaten eingesetzt werden.

10. Verwendung nach einem der Ansprüche 1 bis 9, **dadurch gekennzeichnet, dass** als Zusatzstoffe Substanzen des hyalinen Knorpelgewebes ausgewählt aus Glucosaminsulfat und/oder Chondroitinsulfat eingesetzt werden.

11. Verwendung nach einem der Ansprüche 1 bis 10, **dadurch gekennzeichnet, dass** als Zusatzstoffe Substanzen mit steroidaler oder corticosteroidaler Wirkung ausgewählt aus Glucocorticoiden eingesetzt werden.

**12.** Verwendung nach einem der Ansprüche 1 bis 11, **dadurch gekennzeichnet, dass** als Zusatzstoffe nichtsteroidale Antiphlogistika ausgewählt aus Indometacin, Dichlorphenac oder Salicylsäure eingesetzt werden.

**13.** Verwendung nach einem der Ansprüche 1 bis 12, **dadurch gekennzeichnet, dass** als Zusatzstoffe Analgetika ausgewählt aus Oxicamen, Anilin oder Anthranilsäure eingesetzt werden.

**14.** Verwendung nach einem der Ansprüche 1 bis 13, **dadurch gekennzeichnet, dass** als Zusatzstoffe Substanzen mit hemmender Wirkung auf die Prostaglandinsynthese ausgewählt aus Lipoxygenasehemmern, Cyclooxygenasehemmern und Phospholipase-A2-Hemmern eingesetzt werden.

**15.** Verwendung nach einem der Ansprüche 1 bis 14, **dadurch gekennzeichnet, dass** als Zusatzstoffe Wachstumsfaktoren ausgewählt aus Retinol oder Bone Morphogenetic Proteins (BMP's) eingesetzt werden.

**16.** Verwendung nach mindestens einem der Ansprüche 1 bis 15, **dadurch gekennzeichnet, dass** als Zusatzstoff Vitamine eingesetzt werden.

**17.** Verwendung nach Anspruch 16, **dadurch gekennzeichnet, dass** als Vitamine Vitamin A, C, 312 oder Biotin eingesetzt werden.

**18.** Verwendung nach einem der Ansprüche 1 bis 17, **dadurch gekennzeichnet, dass** als Zusatzstoffe Antioxidantien ausgewählt aus Flavonoiden oder Glutathionen eingesetzt werden.

**19.** Verwendung nach einem der Ansprüche 1 bis 18, **dadurch gekennzeichnet, dass** als Zusatzstoffe Substanzen mit wasserbindenden Eigenschaften ausgewählt aus Harnstoff oder Arginin eingesetzt werden.

## Claims

**1.** Use of hydrauolic acid and/or its salts in combination with lidocaine as well as possibly further additives as intra-articular or intradiscalic formulations for producing a preparation for human and veterinary therapy, the prophylaxis and/or metaphylaxis of degenerative or traumatic joint diseases and joint function problems, whereby hydrauolic acid and/or its salts and lidocaine are bound to each other in chemical or physical form, and in that the release of lidocaine is delayed.

**2.** Use according to Claim 1 for human and veterinary therapy, the prophylaxis and/or metaphylaxis of joint cartilage and cartilage bone defects.

**3.** Use according to at least one of the Claims 1 and 2 for human and veterinary therapy, the prophylaxis and/or metaphylaxis of meniscus and disc diseases.

**4.** Use according to at least one of the Claims 1 to 3 for stabilising and/or regenerating joint structures, in particular the joint cartilage and the meniscus.

**5.** Use according to at least one of the Claims 1 to 4, **characterised in that** hydrauolic acid and/or its salts is used in a concentration of between 0.001 and 5 wt. %.

**6.** Use according to at least one of the Claims 1 to 5, **characterised in that** hydrauolic acid and/or its salts is used in a concentration of between 0.2 and 2 wt. %.

**7.** Use according to at least one of the Claims 1 to 6, **characterised in that** lidocaine is used in a concentration of between 0.001 and 20 wt. %.

**8.** Use according to at least one of the Claims 1 to 7, **characterised in that** lidocaine is used in a concentration of between 0.001 and 5.0 wt. %.

**9.** Use according to one of the Claims I to 8, **characterised in that** radical absorbers used as additives are selected from tocopherol derivatives and/or ascorbic acid derivatives.

**10.** Use according to one of the Claims 1 to 9, **characterised in that** substances of the hyalinic cartilage tissue used as additives are selected from glucosamine sulfate and/or chondroitine sulfate.

**11.** Use according to one of the Claims 1 to 10, **characterised in that** substances with steroidal or corticosteroidal effect used as additives are selected from glucocorticoids.

**12.** Use according to one of the Claims 1 to 11, **characterised in that** non-steroidal antiphlogistica used as additives are selected from indometacin, dichlorophenac, or salicylic acids.

**13.** Use according to one of the Claims 1 to 12, **characterised in that** analgesia used as additives are selected from oxicamen, anilin, or anthranilic acid.

**14.** Use according to one of the Claims 1 to 13, **characterised in that** substances with an inhibiting effect on the prostate gland synthesis used as additives are selected from lipoxy genasis inhibitors, cyclooxy genasis inhibitors, and phospholipase A2 inhibitors.

**15.** Use according to one of the Claims 1 to 14, **characterised in that** growth factors used as additives are selected from retinol or bone morphogenetic proteins (BMPs).

**16.** Use according to one of the Claims 1 to 15, **characterised in that** vitamins are used as additives.

**17.** Use according to Claim 16, **characterised in that** vitamin A, C, 312, or biotin are used as vitamins.

**18.** Use according to one of the Claims 1 to 17, **characterised in that** antioxidants used as additives are selected from flavonoids or glutathions.

**19.** use according to one of the Claims 1 to 18, **characterised in that** substances with water binding characteristics used as additives are selected from urea or arginin.

## Revendications

**1.** Utilisation d'acide hyaluronique et/ou de ses sels en association avec de la lidocaïne ainsi qu'éventuellement d'autres additifs, en tant que formulation intra-articulaire ou intradiscale pour la fabrication d'une préparation destinée à la thérapie, la prophylaxie et/ou la métaphylaxie en médecines humaine et vétérinaire d'arthropathies et de troubles de la fonction articulaire traumatiques ou dégénératifs, l'acide hyaluronique et/ou ses sels et la lidocaïne étant utilisés sous forme physiquement ou chimiquement liée l'un à l'autre, et la lidocaïne étant libérée de façon retardée.

**2.** Utilisation selon la revendication 1, pour la thérapie, la prophylaxie et/ou la métaphylaxie en médecines humaine et vétérinaire de défauts de cartilages articulaires ou d'os cartilagineux.

**3.** Utilisation selon la revendication 1 ou 2 pour la thérapie, la prophylaxie et/ou la métaphylaxie en médecines humaine et vétérinaire des maladies du ménisque et des disques intervertébraux.

**4.** Utilisation selon au moins l'une des revendications 1 à 3, pour la stabilisation et/ou la régénération de structures articulaires, en particulier du cartilage articulaire et du ménisque.

**5.** Utilisation selon au moins l'une des revendications 1 à 4, **caractérisée en ce que** l'acide hyaluronique et/ou ses sels est(sont) utilisé(s) à une concentration comprise entre 0,001 et 5 % en poids.

**6.** Utilisation selon au moins l'une des revendications 1 à 5, **caractérisée en ce que** l'acide hyaluronique et/ou ses sels est(sont) utilisé(s) à une concentration comprise entre 0,2 et 2 % en poids.

**7.** Utilisation selon au moins l'une des revendications 1 à 6, **caractérisée en ce que** la lidocaïne est utilisée à une concentration comprise entre 0,001 et 20 % en poids.

**8.** Utilisation selon au moins l'une des revendications 1 à 7, **caractérisée en ce que** la lidocaïne est utilisée à une concentration comprise entre 0,001 et 5,0% en poids.

**9.** Utilisation selon l'une quelconque des revendications 1 à 8, **caractérisée en ce qu'**on utilise comme additifs des capteurs de radicaux choisis parmi les dérivés de tocophérol et/ou les dérivés d'acide ascorbique.

**10.** Utilisation selon l'une quelconque des revendications 1 à 9, **caractérisée en ce qu'**on utilise comme additifs des substances du tissu cartilagineux hyalin choisies parmi le sulfate de glucosamine et/ou le sulfate de chondroïtine.

**11.** Utilisation selon l'une quelconque des revendications 1 à 10, **caractérisée en ce qu'**on utilise comme additifs des substances à action stéroïdienne ou corticostéroïdienne, choisies parmi les glucocorticoïdes.

**12.** Utilisation selon l'une quelconque des revendications 1 à 11, **caractérisée en ce qu'**on utilise comme additifs des anti-inflammatoires non stéroïdiens choisis parmi l'indométacine, le dichlorphénac et l'acide salicylique.

**13.** Utilisation selon l'une quelconque des revendications 1 à 12, **caractérisée en ce qu'**on utilise comme additifs des analgésiques choisis parmi les oxicams, l'aniline et l'acide anthranilique.

**14.** Utilisation selon l'une quelconque des revendications 1 à 13, **caractérisée en ce qu'**on utilise comme additifs des substances à effet inhibiteur sur la synthèse des prostaglandines, choisies parmi les inhibiteurs de lipoxygénase, les inhibiteurs de cyclo-oxygénase et les inhibiteurs de phospholipase A2.

**15.** Utilisation selon l'une quelconque des revendications 1 à 14, **caractérisée en ce qu'**on utilise comme additifs des facteurs de croissance choisis parmi le rétinol et les protéines morphogénétiques de l'os (BMP).

**16.** Utilisation selon au moins l'une des revendications 1 à 15, **caractérisée en ce qu'**on utilise comme additifs des vitamines.

**17.** Utilisation selon la revendication 16, **caractérisée en ce qu'**on utilise comme vitamines la vitamine A, C, 312 ou la biotine.

**18.** Utilisation selon l'une quelconque des revendications 1 à 17, **caractérisée en ce qu'**on utilise comme additifs des antioxydants, choisis parmi les flavonoïdes ou les glutathions.

**19.** Utilisation selon l'une quelconque des revendications 1 à 18, **caractérisée en ce qu'**on utilise comme additifs des substances à propriétés fixant l'eau, choisies parmi l'urée et l'arginine.

Figur 1

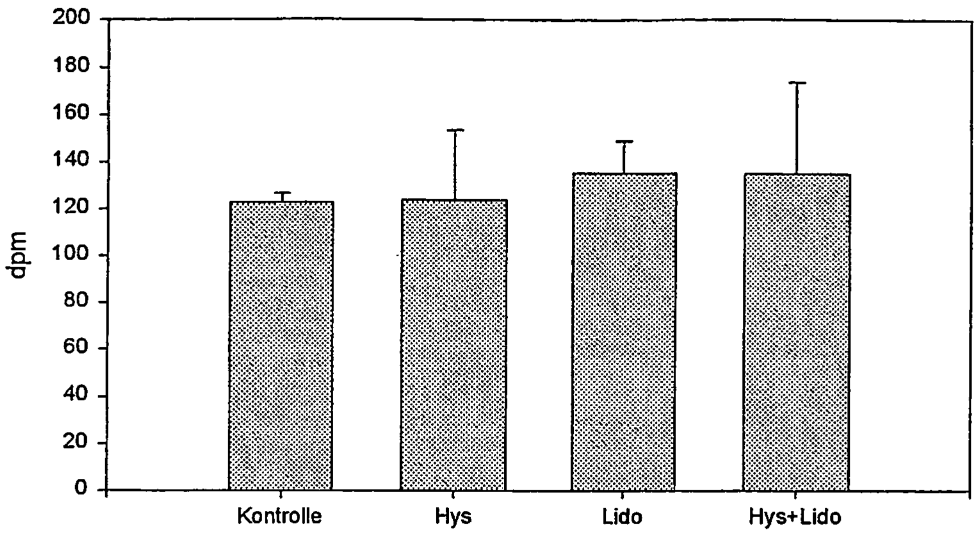
200
180
160
140
120
100
80
60
40
20
0
dpm
Kontrolle
Hys
Lido
Hys+Lido

Figur 2

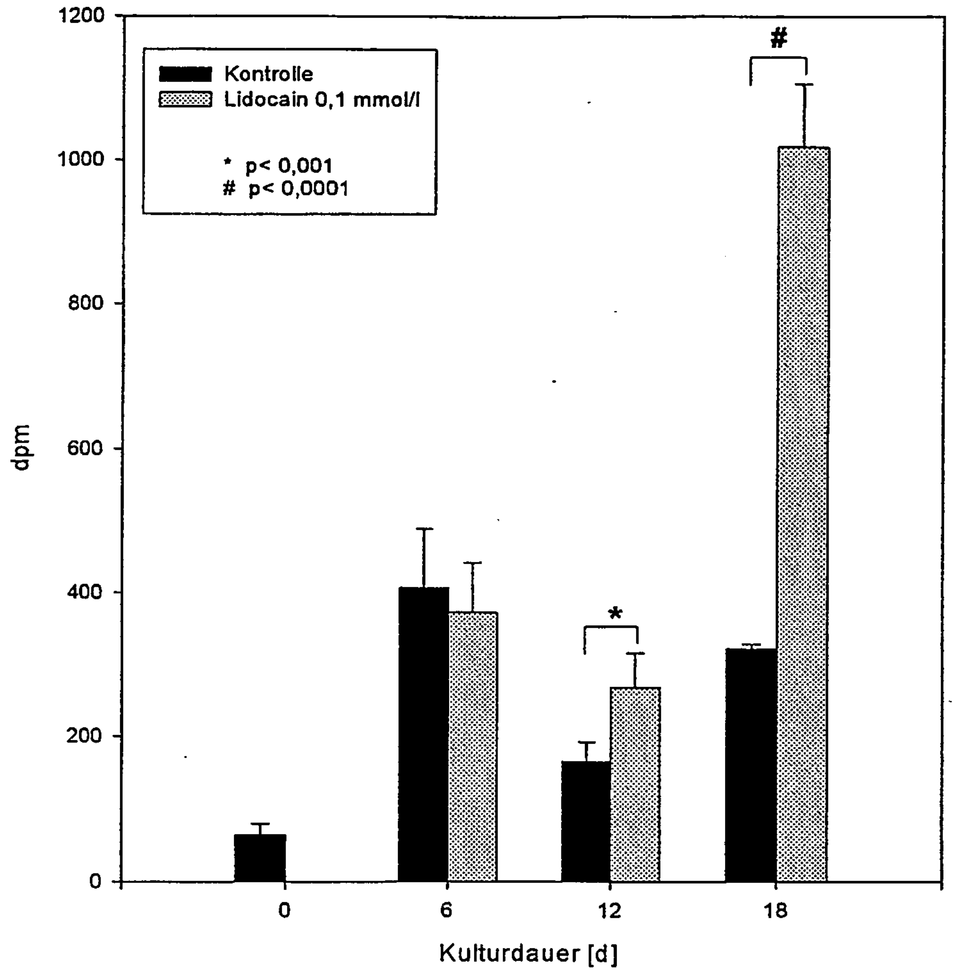
1200
1000
800
600
400
200
0
dpm
Kontrolle
Lidocain 0,1 mmol/l
* p< 0,001
# p< 0,0001
#
*
0
6
12
18
Kulturdauer [d]

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **A. LUSSIER et al.** *J Rheumatol.,* 1996, vol. 23, 1579-1585
- **D. SCALE et al.** *Current Therapeutic Research.,* 1994, vol. 55, 220-232
- **M. WOBIG et al.** *Clinical Therapeutics.,* 1998, vol. 20, 410-4.23
- **K.W.MARSHALL.** *Today's Therapeutic Trends,* 1997, vol. 15, 99-108
- **K.W.MARSHALL.** *Curr. Opin.Rheumatol,* 2000, vol. 12, 468-474
- **J.P.PELLETIER et al.** *J Rheumatol,* 1993, vol. 20, 19-24
- **K.KAWASAKI et al.** *Cell Physiol.,* 1999, vol. 179, 142-148
- **D. WOHLRAB et al.** *hylan news,* 2000, vol. 2, 2-5